**Europäisches Patentamt**

**European Patent Office**

**Office européen des brevets**

(19)

(11) Veröffentlichungsnummer: **0 135 601**
**B1**

(12)

# EUROPÄISCHE PATENTSCHRIFT

(45) Veröffentlichungstag der Patentschrift:
07.12.88

(51) Int. Cl.⁴: **C 07 G 17/00, A 61 K 35/64**

(21) Anmeldenummer: **83109531.0**

(22) Anmeldetag: **24.09.83**

(54) Verfahren zum Herstellen eines Propolis-Extraktes.

(43) Veröffentlichungstag der Anmeldung:
03.04.85 Patentblatt 85/14

(45) Bekanntmachung des Hinweises auf die Patenterteilung:
07.12.88 Patentblatt 88/49

(84) Benannte Vertragsstaaten:
AT BE CH FR GB IT LI NL SE

(56) Entgegenhaltungen:
EP-A- 0 061 508
US-A- 4 382 886
US-A- 4 382 886

Soviet Inventions Illustrated Week E39, 10 November 1982 Sections B04, D23 & SU-A-884 703 (M.V. OGILETS et al.)
Chemical Abstracts Band 72, Nr. 8, 23. Februar 1970, Columbus, Ohio, USA Seite 323, Spalte 2, Abstract Nr. 35688u & SU-A-249 564
Chemical Abstracts Band 98, Nr. 2, 10. Januar 1983, Columbus, Ohio, USA Seite 219, Spalte 1, Abstract Nr. 8087c & HU-A-22 624
Chemical Abstracts Band 93, Nr. 18, 3. November 1980, Columbus, Ohio, USA Seite 355, Spalte 1, Abstract Nr. 173758b & RO-A-67 036
Chemical Abstracts Band 94, Nr. 19, 11. Mai 1981, Columbus, Ohio, USA Seite 225, Spalte 1, Abstract Nr. 151901m & RO-A-69690

(73) Patentinhaber: **Peter O. Glienke + Partner Industrieberatung GmbH, Luisenstrasse 20, D-7570 Baden-Baden 1 (DE)**

(72) Erfinder: **Glienke, Peter O., Eichelbergstrasse 27, D-7570 Baden-Baden 24 (DE)**
Erfinder: **Hehl, Heinrich, Dr., Sandweierer Strasse 18, D-7570 Baden-Baden (DE)**

(74) Vertreter: **Trappenberg, Hans, Postfach 1909, D-7500 Karlsruhe 1 (DE)**

**Beschreibung**

Die Erfindung betrifft ein Verfahren zum Herstellen eines in der Human- und Veterinärmedizin anwendbaren, biologisch verwertbaren Propolis-Extraktes aus Rohpropolis.

Propolis ist ein Harz, das aus einer grossen Menge von verschiedenartigen Stoffen besteht, die von den Bienen in den Bienenstock eingetragen werden.

Mit Propolis verkitten die Bienen ihre Behausung, verstärken ihre Waben und glätten rauhe Teile des Stockes. Ausserdem werden die Waben, wie auch sämtliche Behausungsteile, mit einem dünnen Propolisfilm überzogen, der nach neueren Forschungen dafür verantwortlich ist, dass bei Bienen trotz der dichten Bevölkerung in einem Bienenstock keine Seuchen auftreten.

Von dieser antibakteriellen Eigenschaft ausgehend wurde die Propolis auch schon im Altertum als insbesondere äusserlich anzuwendendes Heilmittel gebraucht. Diesem Gebrauch kommt die wachs- bis harzartige Konsistenz der Propolis entgegen. Für innere Anwendung jedoch sollte die Propolis in Lösung gebracht werden, um sie biologisch verfügbar zu machen.

Zu diesem Zweck wurde bisher Propolis mit Alkohol extrahiert oder durch Wärmebehandlung die wachsartigen Stoffe der Propolis entfernt. Sowohl die Extraktion wie auch die Wärmebehandlung hinterliessen jedoch Stoffe, denen die zuvor zugeschriebenen Heilwirkungen nicht mehr oder nur im geringen Masse zukamen. Insbesondere bei der Wärmebehandlung der Propolis gehen anscheinend für die Heilwirkung notwendige Stoffe verloren beziehungsweise werden so umgewandelt, dass sie biologisch nicht mehr verfügbar sind.

Die Wärmebehandlung wird vermieden und eine besonders schonende Extraktion angewendet bei einem durch die US-A-4 382 886 bekannt gewordenen Verfahren. Ein weiteres, ebenfalls diese Wärmebehandlung vermeidendes Verfahren ist bekannt geworden durch einen Aufsatz in «Soviet Inventions Illustrated Week E39, 10. November 1982, Sections B04».

Die Erfindung gibt ein neues Verfahren an, wie die Propolis in ein biologisch verfügbares und verwertbares Propolis-Extrakt mit hohem Wirkstoffgehalt gebracht werden kann, aus dem sodann sowohl Lösungen wie auch Salben etc. hergestellt werden können.

Dieses Verfahren zum Herstellen eines in der Human- und Veterinärmedizin anwendbaren, biologisch verwertbaren Propolis-Extraktes aus Roh-Propolis, mit den Verfahrensschritten Extraktion und/oder Perkolation mit einem organischen Lösungsmittel oder mit einem Gemisch aus organischen Lösungsmitteln und Wasser, vorzugsweise mit einem Gemisch aus polaren organischen Lösungsmitteln und Wasser, Entfernen des Lösungsmittels durch Vakuum-Destillation sowie Zugabe eines organischen, höhere Fettsäuren und mehrwertige Alkohole enthaltenden Emulgators als Lösungsvermittler ist dadurch gekennzeichnet, dass der Emulgator/Lösungsvermittler gleichzeitig mit dem Entfernen des Lösungsmittels oder Lösungsmittelgemisches zugegeben und als Emulgator/Lösungsvermittler ein Polyoxyäthylenderivat von partiellen Estern des Glycerins mit natürlichen Fettsäuren und/oder ein Teilester des Glycerins, des Äthylenglykols oder des 1,2-Propylenglykols mit natürlichen Fettsäuren oder ein Gemisch und/oder ein Umsetzungsprodukt von Glycerinestern, insbesondere der Ricinolsäure und der Stearinsäure und von Polyäthylenglykolen eingesetzt wird.

Überraschenderweise hat sich gezeigt, dass bei Behandlung der Rohpropolis nach dieser Anweisung sich ein bioverfügbares Propolisextrakt mit äusserst hohem Flavonoidgehalt ergibt. Grundkörper dieser Flavonoide ist das farblose, fast wasserunlösliche Flavon, das in einem Belag auf den Blättern, Blütenstielen und Knospen verschiedener Pflanzen vorkommt und von den Bienen gesammelt wird. Je nach dem Oxidationsgrad dieser Flavone und der Struktur des heterocyclischen Ringsystems werden diese Flavone nochmals unterteilt in Isoflavone, Flavanone und Isoflavanone sowie in Flavanole und Flavanonole. Diesen Flavonoiden können, wie die Erfahrung gezeigt hat, die ausserordentlichen Heilwirkungen der Propolis zugeschrieben werden.

Der erfindungsgemässe Emulgator/Lösungsvermittler hat nun überraschenderweise die Eigenschaft, diese Flavonoide aus dem Rohpropolis herauszulösen, zusammen mit einigen methylierten Verbindungen, die – das kann zumindest angenommen werden – mit zum Heilwert der Propolis beitragen. Dieser Propolisextrakt trägt nun die gesamten heilwirksamen Stoffe der Propolis in einer biologisch verfügbaren beziehungsweise biologisch verwertbaren Form, wobei die Heilstoffe praktisch vollkommen im Magen/Darmtrakt und/oder über die Schleimhäute aufgenommen werden.

Damit liegt erstmals ein Propolis-Extrakt vor, der sowohl in der Human- wie auch in der Veterinärmedizin direkt, wie auch in Verbindung mit anderen Stoffen, angewendet werden kann.

In der Chemie ist die Extraktion mit organischen Lösungsmitteln, auch mit Gemischen aus organischen Lösungsmitteln und Wasser, selbstverständlich bekannt, jedoch ist der überraschende Effekt, der sich nach der erfindungsgemässen Anweisung ergibt, nämlich die einwandfreie Extraktion der Rohpropolis, so, dass sich gerade die heilwirksamen Stoffe in einer Emulsion standardisieren lassen, noch nicht bekannt.

Zweckmässigerweise wird dem Lösungsmittel/Wassergemisch zusätzlich bis zu 10 Gew.-% N-Methylpyrolidon zugesetzt. Auch können dem Emulgator/Lösungsvermittler zusätzlich Polyäthylenglykole, Propylenglykole und/oder Zuckeralkohole (Hexite) wie auch ein Antioxidans, beispielsweise alpha-Tocopherol (D1-alpha-Tocopherol) (Vitamin E) oder ein Kombinations-Antioxidans beigefügt werden.

Liegt das extrahierte Produkt vor, so kann selbstverständlich ohne Schwierigkeiten ein Gewichtsstandard und ein biologischer Standard der Hauptwirksubstanz herbeigeführt werden.

Das erfindungsgemässe Verfahren soll an folgendem Ausführungsbeispiel erläutert werden:

1,0 kg Rohpropolis mit einem durchschnittlichen Gesamtflavonoidgehalt von 4 Gew.-% werden fein zerkleinert und in einem Perkolator mit 5,0 kg Lösemittelgemisch, bestehend aus 70 Gew.-% Aceton und 30 Gew.-% Wasser extrahiert. Das so erhaltene Propolis-Perkolat mit einem Feststoffgehalt von 10 Gew.-% wird in einem Rotationsverdampfer unter gleichzeitiger Zugabe von 2,0 kg eines Emulgators, bestehend aus dem Umsetzungsprodukt der Ricinolsäure mit Äthylenoxid, auf Gewichtskonstanz von 2,5 kg eingeengt.

Der so gewonnene Propolis-Extrakt enthält 20 Gew.-% standardisiertes Propolis und kann nun beliebig mit Wasser oder mit Alkohol usw. verdünnt werden. Der Extrakt kann auch leicht in Salben, Cremes oder Gelee eingearbeitet werden.

Die Überprüfung des Gesamt-Flavonoidgehaltes des oben beschriebenen Extraktes ergab, dass nach dem angewendeten Extraktions- und Destillationsverfahren eine Ausbeute in Gesamt-Flavonoide von 95% (bezogen auf den Ausgangs-Flavonoidgehalt) gegeben war. Dieses Propolis-Standardextrakt mit 20 Gew.-% standardisiertem Propolis kann beispielsweise in der Humanmedizin wie folgt eingesetzt werden:

1,0 kg Propolis-Standardextrakt werden mit 1,0 kg 90%-igem Alkohol versetzt. Die so erhaltene alkoholische Propolislösung enthält 10% standardisierten Propolis-Extrakt und findet Verwendung als Tropfen zur vorbeugenden Behandlung von Erkältungskrankheiten, insbesondere von Infektionen beziehungsweise Infektionsvorbeugung im Bereich der oberen Atemwege oder dient dem Aufbau eines Mund- und Rachensprays mit bakterizider und infektionshemmender Wirkung.

## Patentansprüche

1. Verfahren zum Herstellen eines in der Human- und Veterinärmedizin anwendbaren, biologisch verwertbaren Propolis-Extraktes aus Roh-Propolis, mit den Verfahrensschritten Extraktion und/oder Perkolation mit einem organischen Lösungsmittel oder mit einem Gemisch aus organischen Lösungsmitteln und Wasser, vorzugsweise mit einem Gemisch aus polaren organischen Lösungsmitteln und Wasser, Entfernen des Lösungsmittels durch Vakuum-Destillation sowie Zugabe eines organischen, höhere Fettsäuren und mehrwertige Alkohole enthaltenden Emulgators als Lösungsvermittler, dadurch gekennzeichnet, dass die Zugabe von Emulgator/Lösungsvermittler gleichzeitig mit dem Entfernen des Lösungsmittels oder Lösungsmittelgemisches erfolgt und dass der Emulgator/Lösungsvermittler ein Polyoxyäthylenderivat von partiellen Estern des Glycerins mit natürlichen Fettsäuren und/oder ein Teilester des Glycerins, des Äthylenglykols oder des 1,2-Propylenglykols mit natürlichen Fettsäuren oder ein Gemisch und/oder ein Umsetzungsprodukt von Glycerinestern, insbesondere der Ricinolsäure und der Stearinsäure und von Polyäthylenglykolen ist.

2. Verfahren nach Anspruch 1, dadurch gekennzeichnet, dass dem Lösungsmittel/Wassergemisch zusätzlich bis zu 10 Gew.-% N-Methylpyrolidon zugesetzt sind.

3. Verfahren nach Anspruch 1, dadurch gekennzeichnet, dass zum Emulgator/Lösungsvermittler zusätzlich Polyäthylenglykole, Propylenglykole und/oder Zuckeralkohole (Hexite) beigefügt sind.

4. Verfahren nach Anspruch 1, dadurch gekennzeichnet, dass zum Emulgator/Lösungsvermittler ein Antioxidans, beispielsweise alpha-Tocopherol (D1-alpha-Tocopherol) (Vitamin E) oder ein Kombinations-Antioxidans beigefügt ist.

## Claims

1. A method of production from raw propolis of a biologically utilizable propolis extract applicable in human and veterinary medicine, by the steps of the method of extraction and/or percolation with an organic solvent or with a mixture of organic solvents and water, preferably with a mixture of polar organic solvents and water, removal of the solvent through vacuum distillation as well as the addition of an organic emulsifying agent containing higher fatty acids and polyhydric alcohols as a solubilizer, characterized in that the addition of emulsifying agent/solubilizer is effected simultaneously with the removal of the solvent or mixture of solvents and that the emulsifying agent/solubilizer is a polyoxyethylene derivative from partial esterification of glycerine with natural fatty acids and/or a partial ester of glycerine, ethylene glycol or 1,2-propylene glycol with natural fatty acids or a mixture and/or a conversion product of glycerine esterification, in particular of ricinoleic acid and of stearic acid and of polyethyleneglycols.

2. A method as in Claim 1, characterized in that to the solvent/water mixture is added in addition up to 10% by wt. of N-methylpyrolidone.

3. A method as in Claim 1, characterized in that to the emulsifying agent/solubilizer are added in addition polyethylene glycols, propylene glycols and/or sugar alcohols (hexites).

4. A method as in Claim 1, characterized in that to the emulsifying agent/solubilizer is added an antioxidant, for example, alpha-tocopherol (D1-alpha-tocopherol) (vitamin E) or a combination-antioxidant.

## Revendications

1. Procédé pour fabriquer, à partir de la propolis brute, un extrait de propolis biologiquement exploitable et utilisable en médecines humaine et vétérinaire, comprenant les étapes opératoires d'une extraction et/ou d'une percolation avec un solvant organique ou avec un mélange de solvants organiques et d'eau, de préférence avec un mélange de solvants organiques polaires et d'eau, d'une élimination du solvant par distillation sous vide, ainsi que de l'adjonction, en tant qu'agent de solubilisation, d'un émulsifiant organique renfermant des acides gras supérieurs et des alcools polyvalents, caractérisé par le fait que l'adjonction d'émulsifiant/agent de solubilisation a lieu simultanément à l'élimination du solvant ou du mélange de solvant; et par le fait que l'émulsifiant/agent de solubilisation est un dérivé polyoxyéthylène d'esters partiels de la glycérine avec des acides gras naturels et/ou un ester partiel de la glycérine, de l'éthylène-glycol ou du 1,2-propylène-glycol avec des acides gras naturels ou bien un mélange et/ou un produit de transformation d'esters de glycérine, en particulier de l'acide ricinoléique et de l'acide stéarique et de polyéthylènes-glycols.

2. Procédé selon la revendication 1, caractérisé par le fait qu'on adjoint additionnellement jusqu'à 10% en poids de N-méthylpyrolidone au mélange solvants/eau.

3. Procédé selon la revendication 1, caractérisé par le fait que des polyéthylènes-glycols, des propylènes-glycols et/ou des alcools de sucre (hexites) sont ajoutés additionnellement à l'émulsifiant/agent de solubilisation.

4. Procédé selon la revendication 1, caractérisé par le fait qu'un anti-oxydant, par exemple de l'alpha-tocophérol (D1-alpha-tocophérol) (vitamine E), ou bien un anti-oxydant de combinaison, est ajouté à l'émulsifiant/agent de solubilisation.